(19) **Europäisches Patentamt / European Patent Office / Office européen des brevets**

(11) **EP 4 643 766 A1**

(12) # DEMANDE DE BREVET EUROPEEN

(43) Date de publication:
05.11.2025 Bulletin 2025/45

(21) Numéro de dépôt: **25172762.4**

(22) Date de dépôt: **25.04.2025**

(51) Classification Internationale des Brevets (IPC):
**A61B 5/021** (2006.01)    **A61B 5/00** (2006.01)

(52) Classification Coopérative des Brevets (CPC):
**A61B 5/02108; A61B 5/6831; A61B 5/7264;**
A61B 5/02416; A61B 5/02438; A61B 5/681;
A61B 5/6822; A61B 5/6823; A61B 5/6824;
A61B 2562/0204

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH LA MA MD TN**

(30) Priorité: **30.04.2024 FR 2404510**

(71) Demandeur: **Commissariat à l'Energie Atomique et aux Energies Alternatives**
**75015 Paris (FR)**

(72) Inventeurs:
- **VASSEUR, Clément**
  **38054 Grenoble cedex 09 (FR)**
- **VILLENEUVE, Emma**
  **38054 Grenoble cedex 09 (FR)**
- **BEDNAREK, Xavier**
  **38054 Grenoble cedex 09 (FR)**

(74) Mandataire: **INNOV-GROUP**
**209 Avenue Berthelot**
**69007 Lyon (FR)**

(54) **DISPOSITIF ET PROCÉDÉ DE MESURE D'UNE PRESSION ARTÉRIELLE**

(57) Procédé de détermination d'une pression artérielle d'un utilisateur, en mesurant un paramètre suivant une variation monotone durant une systole, le procédé comportant :
- a) au cours d'au moins un cycle cardiaque, mesure (110) proximale du paramètre à une première distance du cœur et mesure (120) distale du paramètre, à une deuxième distance du cœur, la deuxième distance étant supérieure à la première distance ;
- b) à partir de chaque mesure, estimation (130) d'une caractéristique représentative de la variation du paramètre durant la systole à chaque distance;
- c) comparaison (140) de chaque caractéristique (VDMS);
- d) détermination (150) de la pression artérielle (PAM) en fonction de la comparaison résultant de l'étape c), à l'aide d'un modèle de calibration.

Fig. 4

**Description**

**DOMAINE TECHNIQUE**

Le domaine technique de l'invention est la mesure de la pression artérielle.

**ART ANTERIEUR**

La plupart des dispositifs permettant de mesurer la pression artérielle utilisent un capteur de pression couplé à un brassard de compression disposé sur un membre, généralement un bras. La caractérisation de la pression artérielle est effectuée en mesurant la pression exercée par le brassard en un ou plusieurs instants caractéristiques. Le capteur de pression est sensible aux battements cardiaques et à leur amplitude. En général, les dispositifs sont disposés autour de l'artère brachiale.

Dans les tensiomètres grand public, un capteur de pression détermine la pression d'air dans le brassard. La compression du brassard est effectuée de manière à obtenir une occlusion artérielle. Lors de la déflation ou de l'inflation du brassard, des oscillations de pression apparaissent. Les oscillations augmentent jusqu'à atteindre, transitoirement, une amplitude maximale. A cet instant la pression dans le brassard est considérée égale à la pression artérielle moyenne dans l'artère brachiale. A partir de l'amplitude maximale détectée, les instants correspondant respectivement aux pressions artérielles systolique et diastolique sont estimés sur la base de lois empiriques. La pression artérielle moyenne est donc une grandeur pouvant être aisément mesurée par un dispositif grand public. A partir ce cette mesure, la pression artérielle systolique et la pression artérielle diastolique peuvent être calculées.

Un inconvénient des tensiomètres précédemment décrit est la nécessité d'obtenir une compression suffisante pour obtenir une occlusion artérielle. Si l'inconfort est acceptable pour des mesures ponctuelles, la compression est difficilement acceptable pour un suivi en continu de la pression artérielle.

Des méthodes de mesure ont été développées, qui ne nécessitent pas une compression d'un membre. Il s'agit de méthodes de mesure « sans brassard », ou « cuffless ». On connaît par exemple le recours à l'équation de Bramwell-Hill, qui permet d'estimer une variation de pression artérielle à partir d'une vitesse d'onde de pouls estimée en effectuant une détection d'une onde de pouls en deux zones corporelles différentes, par exemple au niveau de l'aorte et du poignet, ou au niveau de la carotide et de l'artère fémorale. La vitesse d'onde de pouls correspond à la distance entre les deux zones divisées par l'écart temporel entre les deux ondes de pouls détectées. Cependant, ce type de méthode nécessite une synchronisation temporelle, de façon que l'écart temporel soit déterminé pour un même battement cardiaque. La synchronisation temporelle peut nécessiter une autre mesure par exemple une mesure électrique de type ECG (Electrocardiogramme).

La publication Mukkamala R et al « Towards ubiquitous blood pressure monitoring via pulse transit time : theory and practice", IEEE transactions on biomedical engineering, vol. 62, n°8, 2015-08-01, décrit les principes de mesure d'un pulse transit time (PTT), par exemple par couplage entre ECG et PPG (photoplethysmographie). La détermination du PTT est basée sur une détection d'instants remarquables résultant de chaque modalité utilisée.

Il est également possible d'estimer la pression artérielle par une analyse de la forme d'une onde de pouls. Mais ce type de méthode est relativement complexe à mettre en œuvre, car dépendant de paramètres physiologiques comme le volume du ventricule gauche, la résistance vasculaire systémique ou la compliance vasculaire.

La systole correspond à la contraction du ventricule gauche du cœur pour éjecter le sang dans le réseau artériel. Au cours d'un cycle cardiaque, c'est au cours de la systole que la pression atteint un niveau maximal, désigné pression systolique.

L'article Obata Y, Mizogami M, Singh S, Nyhan D, Berkowitz DE, Steppan J, Barodka V. "Ejection time: influence of hemodynamics and site of measurement in the arterial tree." Hypertens Res. 2017 Sep;40(9):811-818 établit un lien entre la différence de la durée de la systole et des caractéristiques vasculaires.

L'article Trian JE. "Arterial Blood-Flow Acceleration Time on Doppler Ultrasound Waveforms: What Are We Talking About?" établit un lien entre la forme du pic systolique, y compris le temps de montée systolique, également désigné temps d'accélération, et une sténose ou, de façon plus générale, une artériopathie périphérique. Cet article décrit une estimation du temps de montée systolique à partir de vélocimétrie Doppler.

Les inventeurs proposent une méthode permettant une estimation d'une pression artérielle simple à mettre en œuvre, sans nécessiter une compression d'une artère. La méthode peut être mise en œuvre à l'aide d'un dispositif compact. Elle est bien adaptée à une mesure en continu de la pression artérielle.

**EXPOSE DE L'INVENTION**

Un premier objet de l'invention est un procédé de détermination d'une pression artérielle d'un utilisateur, en mesurant un paramètre suivant une variation monotone durant une systole, le procédé comportant :

- a) au cours d'au moins un cycle cardiaque, mesure proximale du paramètre à une première distance du cœur et mesure distale du paramètre à une deuxième distance du cœur, la deuxième distance étant supérieure à la première distance ;
- b) à partir de chaque mesure, estimation d'une caractéristique représentative de la variation du paramètre durant la systole à chaque distance;
- c) comparaison de chaque caractéristique ;
- d) détermination de la pression artérielle, en fonction de la comparaison résultant de l'étape c), à l'aide d'un modèle de calibration.

La caractéristique représentative de la variation du paramètre durant la systole peut comporter une durée de la variation monotone du paramètre au cours de la systole.

La caractéristique représentative de la variation du paramètre durant la systole peut comporter une pente de la variation monotone du paramètre au cours de la systole.

Selon une possibilité :

- les étapes a) et b) sont mises en œuvre lors de plusieurs battements cardiaques ;
- la caractéristique représentative de la variation du paramètre durant la systole comporte une durée moyenne de la variation monotone du paramètre au cours de la systole desdits battements cardiaque.

Selon une possibilité :

- les étapes a) et b) sont mises en œuvre lors de plusieurs battements cardiaques ;
- la caractéristique représentative de la variation du paramètre durant la systole comporte une pente moyenne ou maximale de la variation monotone du paramètre au cours de la systole desdits battements cardiaque.

Le paramètre peut être choisi parmi :

- une intensité d'une lumière rétrodiffusée ou transmise par une artère ;
- une intensité d'une onde acoustique réfléchie par une artère ;
- une vibration induite par la systole ;
- une impédance électrique induite par la systole.

L'étape d) peut comporter le recours à un modèle de calibration analytique.

Le modèle de calibration peut être un algorithme d'intelligence artificielle supervisé.

Selon une possibilité, chaque mesure est réalisée en appliquant un capteur face et de préférence contre le corps de l'utilisateur.

Un deuxième objet de l'invention est un dispositif d'estimation d'une pression artérielle d'un utilisateur, comportant :

- deux capteurs, configurés pour être appliqués face à une artère de l'utilisateur, et configurés pour mesurer un paramètre de l'utilisateur, le paramètre suivant une variation monotone durant une systole;
- une unité de traitement, destinée à mettre en œuvre les étapes b), c) et d) d'un procédé selon le premier objet de l'invention à partir du paramètre mesuré par chaque capteur.

Chaque capteur peut être choisi parmi : un capteur acoustique, un capteur optique, un capteur tonométrique ou un capteur d'impédance électrique, ou un capteur électromécanique. L'invention sera mieux comprise par les exemples détaillés donnés par la suite, illustrés par les figures suivantes :

## <u>FIGURES</u>

Les figures 1A et 1B schématisent un premier mode de réalisation d'un dispositif selon l'invention.
La figure 1C représente un autre mode de réalisation de dispositif selon l'invention.
La figure 2 montre la position d'une zone corporelle proximale et d'une zone corporelle distale.
La figure 3 schématise deux temps de montée systoliques correspondant respectivement à une zone corporelle proximale et une zone corporelle distale.
La figure 4 schématise les principales étapes d'un procédé selon l'invention.

La figure 5A décrit des pics systoliques respectivement mesurés au niveau d'une zone corporelle proximale et d'une zone corporelle distale.

La figure 5B représente des points fiduciels sur un pic systolique.

La figure 6A représente un premier modèle de calibration, basé sur une différence de temps de montée systolique.

La figure 6B représente un deuxième modèle de calibration, basé sur une différence de pentes de pics systoliques.

La figure 7A représente un mode de réalisation dans lequel on détermine une moyenne de mesures effectuées sur une zone corporelle proximale et sur une zone corporelle distale.

La figure 7B représente un mode de réalisation dans lequel on effectue des interpolations entre des points de mesure.

La figure 7C représente un mode de réalisation dans lequel on effectue des appariements entre des points de mesure.

## EXPOSE DE MODES DE REALISATION PARTICULIERS

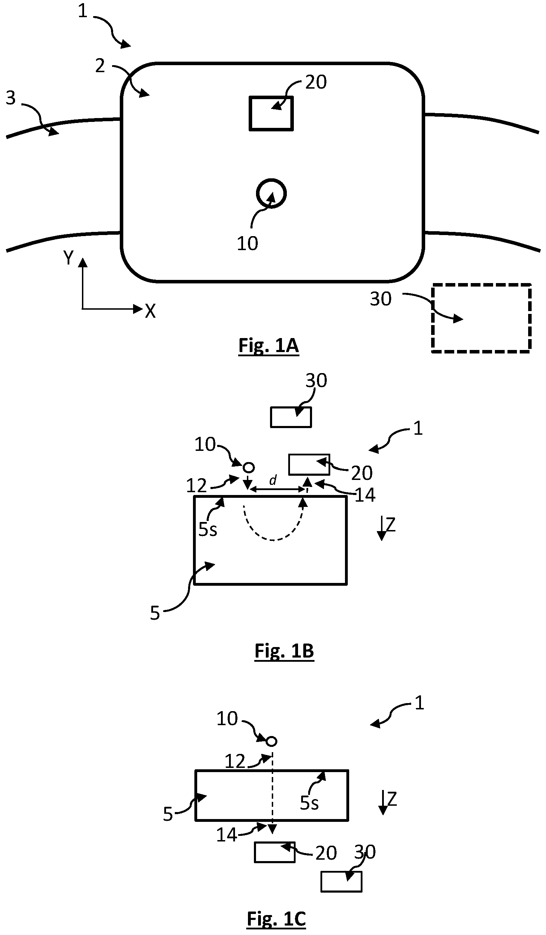

La figure 1A représente un exemple d'un dispositif 1 permettant une mise en œuvre de l'invention. Le dispositif comporte deux modules de mesures 2. Chaque module de mesure 2 comporte un capteur 20, configuré pour détecter un paramètre physiologique représentatif d'une dimension ou d'un volume d'une artère, en différents instants. L'utilisateur peut être un être humain ou un animal vivant. Dans le mode de réalisation décrit en lien avec les figures 1A à 1C, le dispositif 1 met en œuvre un capteur optique.

Chaque module de mesure est destiné à être appliqué contre le corps de l'utilisateur, par exemple contre la peau de l'utilisateur, sans pénétrer à l'intérieur du corps. Chaque module de mesure n'est pas invasif.

Le dispositif 1 est destiné à être appliqué face, et de préférence contre une zone corporelle 5 de l'utilisateur, ou à une faible distance de cette dernière, par exemple inférieure à 5 mm. Le dispositif est de préférence disposé à l'extérieur du corps. La zone corporelle 5 peut par exemple être située au niveau d'un cou ou d'une épaule ou d'un poignet ou d'un doigt de l'utilisateur. Dans l'exemple représenté, le dispositif 1 est relié à un lien 3, de type bracelet, de façon à être fixé contre un poignet. Le dispositif 1 peut être appliqué contre toute autre zone corporelle suffisamment vascularisée : ventre, poitrine, lobe de l'oreille, cuisse, cheville, jambe, ces exemples n'étant pas limitatifs.

Chaque module 2 comporte une source de lumière 10, configurée pour émettre un faisceau lumineux incident 12 vers la zone corporelle adressée face à laquelle la source de lumière est disposée. Le faisceau lumineux incident 12 se propageant vers la zone corporelle 5, selon un axe de propagation Z. Les photons du faisceau lumineux incident 12 pénètrent dans la zone corporelle 5 et une partie d'entre eux est rétrodiffusée, par exemple selon une direction parallèle à l'axe de propagation Z, dans un sens opposé à ce dernier. Les photons rétrodiffusés constituent un rayonnement rétrodiffusé 14. Le rayonnement rétrodiffusé 14 peut être détecté par un photodétecteur 20, placé en regard d'une surface 5s de la zone corporelle. Le photodétecteur 20 peut être configuré de façon à détecter un rayonnement rétrodiffusé émanant de la zone corporelle à une distance d, dite distance de rétrodiffusion, généralement non nulle et inférieure à quelques millimètres, typiquement inférieure à 15 mm ou 10 mm. Le photodétecteur 20 permet de mesurer l'intensité du rayonnement rétrodiffusé.

La source de lumière 10 peut être une LED (Light Emitting Diode - Diode électroluminescente), dont la bande spectrale d'émission s'étend dans le visible ou l'infra-rouge. De préférence, la largeur de la bande spectrale d'émission est inférieure à 100 nm. Le photodétecteur 20 peut être une photodiode. Le signal détecté par le photodétecteur est un signal de photoplethysmographie (PPG).

La figure 1B schématise les principaux composants du dispositif 1 dans un plan perpendiculaire à la surface 5s de la zone corporelle. La flèche courbe en pointillés représente un trajet optique de photons émis par la source de lumière, dans la zone corporelle analysée.

Le dispositif optique 1 comporte une unité de traitement 30 configurée pour traiter un signal détecté par le photodétecteur 20. L'unité de traitement 30 est reliée à une mémoire 32, dans laquelle sont stockées des instructions pour mettre en œuvre le procédé décrit par la suite. L'unité de traitement peut comporter un microprocesseur.

Selon une alternative, représentée sur la figure 1C, la zone corporelle 5 s'étend entre la source de lumière 10 et le photodétecteur 20. Selon une telle configuration, dite en transmission, le photodétecteur 20 mesure une intensité des photons ayant traversé la zone corporelle 5. Une telle configuration suppose que la zone corporelle soit suffisamment fine pour qu'une quantité suffisante de photons émane du milieu et soit détectée par le photodétecteur. Il peut par exemple s'agir du lobe d'une oreille ou du doigt.

Quel que soit le mode de réalisation, le photodétecteur 20 est agencé pour mesurer une intensité d'un faisceau de lumière formé par des photons s'étant propagés à travers la zone corporelle 5 : il s'agit soit de photons rétrodiffusés, soit de photons ayant traversé la zone corporelle. Dans la suite de la description, on se base sur la configuration en réflexion représentée sur la figure 1B.

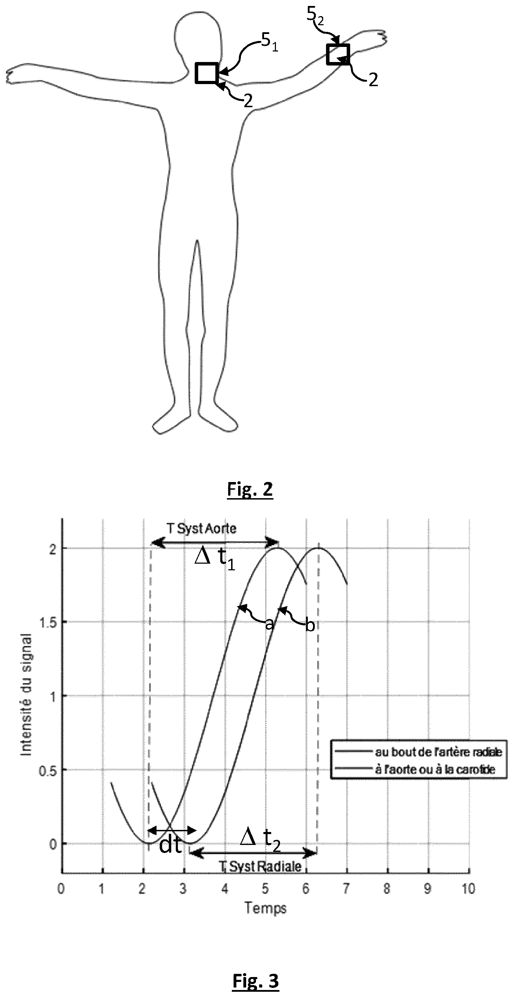

Chaque module 2 du dispositif est appliqué simultanément au niveau d'une zone corporelle proximale $5_1$ et d'une zone corporelle distale $5_2$, comme représenté sur la figure 2. La zone corporelle proximale s'étend à une première distance du cœur. La zone corporelle distale s'étend à une deuxième distance du cœur, la deuxième distance étant supérieure à la première distance. Ainsi, la zone corporelle proximale $5_1$ est plus proche du cœur que la zone corporelle distale $5_2$. De

préférence, l'écart entre la première distance et la deuxième distance est supérieur à 10 cm, voire 20 cm, voire 30 cm. On obtient ainsi une mesure proximale, au niveau de la zone proximale $5_1$, et une mesure distale, au niveau de la zone distale $5_2$.

De préférence, la zone proximale est située à proximité du cœur, par exemple au niveau de l'aorte ou de la carotide. Ainsi, la zone proximale correspond au cou ou au torse de l'utilisateur.

La zone distale peut être située sur un membre, par exemple au niveau d'un poignet ou d'une épaule ou d'une cuisse ou d'un pied. De préférence, la zone distale est disposée au niveau d'une extrémité d'un membre, par exemple au niveau d'une cheville ou d'un poignet.

La figure 3 montre le principe sur lequel est basé l'invention. Sur la figure 3, on a représenté une évolution temporelle théorique de l'intensité d'un signal PPG (axe des ordonnées - unité arbitraire), présentant une partie croissante monotone sur la durée de la montée systolique, mesuré par un dispositif tel que décrit en lien avec les figures 1A à 1C. L'axe des abscisses correspond au temps. Sous l'effet d'une variation du volume sanguin au cours de la systole, le signal détecté par le capteur augmente jusqu'à l'atteinte d'une intensité correspondant à la pression systolique. Sur la figure 3, on a représenté les signaux respectivement mesurés au niveau de la zone proximale (carotide - courbe a) et de la zone distale (artère radiale au niveau du poignet - courbe b), et correspondant à un même battement. On observe un décalage temporel dt entre le début de chaque pic, qui est due à l'écart entre la première distance et la deuxième distance.

Sur la figure 3, on a représenté les temps de montée systoliques $\Delta t_1$ et $\Delta t_2$ déterminés respectivement au niveau de la zone proximale et de la zone distale. Chaque temps de montée correspond à l'augmentation de la pression sous l'effet de la systole. On observe que $\Delta t_2 < \Delta t_1$. L'invention tire profit du constat, réalisé par les inventeurs, que l'écart temporel entre les deux temps de montée VDMS= $\Delta t1$-$\Delta t2$ dépend de la pression artérielle de l'utilisateur. VDMS signifie Variation de la Durée de la Montée Systolique.

En effet, plus la pression artérielle moyenne est élevée, plus une artère est rigide, et plus la vitesse d'onde de pouls est élevée. De ce fait, la vitesse d'onde de pouls est plus élevée pour une pression élevée que pour une pression faible. Au cours d'un cycle cardiaque, la pression élevée, qui correspond à la fin de la systole, se propage plus vite qu'une pression faible, qui correspond au début de la systole. De ce fait, l'écart temporel entre le début et la fin de chaque pic systolique tend à se réduire avec la distance de la propagation de l'onde de pouls. Il est donc plus faible lorsqu'on s'éloigne du cœur. Pour les mêmes raisons, la pente du pic systolique augmente lorsqu'on s'éloigne du cœur. Plus la pression artérielle est forte, plus la VDMS est faible. Par pression artérielle, on entend ici la pression artérielle moyenne PAM.

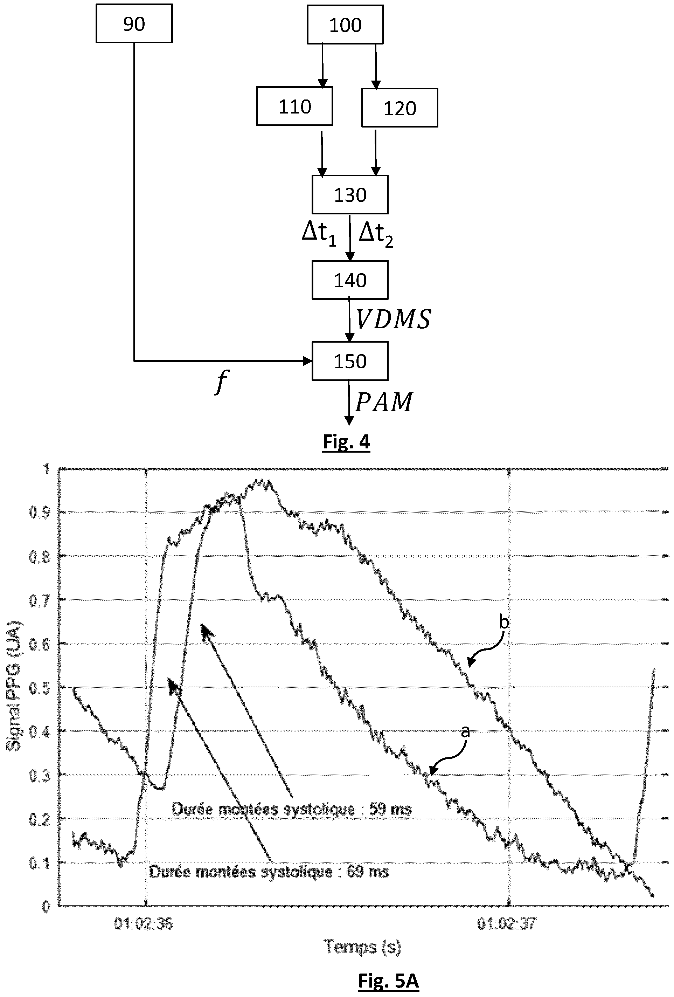

La figure 4 montre les principales étapes d'un procédé de détermination de la pression artérielle d'un utilisateur.

Etape 100 : application d'un dispositif de mesure contre le corps de l'utilisateur, un premier module 2 étant appliqué au niveau d'une zone corporelle proximale et un deuxième module étant appliqué au niveau d'une zone corporelle distale telles que précédemment décrites. Le dispositif de mesure est par exemple un dispositif tel que décrit en lien avec les figures 1A et 1B, sachant qu'il peut s'agir d'un autre type de dispositif, comme décrit par la suite.

Etape 110 : mesure d'un paramètre dépendant d'un volume de sang artériel, au niveau de la zone proximale $5_1$. Le paramètre peut être une intensité d'un faisceau lumineux rétrodiffusé ou transmis par l'artère, ou une intensité d'une onde acoustique réfléchie par l'artère, ou une variation de pression mesurée au niveau de la peau ou une impédance électrique mesurée au niveau de la peau.

Etape 120 : mesure d'un paramètre dépendant d'un volume de sang artériel au niveau de la zone distale $5_1$. De préférence, le paramètre mesuré au niveau de la zone distale est de la même nature que le paramètre mesuré au niveau de la zone proximale, mais cette condition n'est pas nécessaire.

Les étapes 110 et 120 sont répétées selon une fréquence d'acquisition de préférence supérieure à une fréquence minimale définie par la suite.

Etape 130 : détermination d'une caractéristique de la montée systolique en chaque zone corporelle. La caractéristique peut être le temps de montée systolique $\Delta t_1$ et $\Delta t_2$ respectivement au niveau de la zone corporelle proximale et de la zone corporelle distale. D'autres caractéristiques de la montée systolique peuvent être déterminées, par exemple la pente du pic systolique . Cette étape est mise en œuvre par l'unité de traitement 30, à partir des mesures résultant des étapes 110 et 120. De préférence les étapes 110, 120 et 130 sont mises en œuvre en différents battements successifs. Lors de chaque systole, chaque mesure présente un front, montant ou descendant, dû à l'augmentation du volume de sang dans l'artère. Le pic systolique est identifié avec un filtrage du bruit du signal (exemple passe bas), puis avec un algorithme de détection de pic classique par exemple basé sur un changement de signe de la dérivée. Le pic systolique est aisément identifié car il s'agit du pic présentant la plus grande amplitude, et survenant selon une période régulière . Une caractéristique du pic systolique, par exemple la durée du temps de montée, peut être déterminée lors de chaque battement, et cela à partir du paramètre mesuré par la mesure distale et par la mesure proximale.

La figure 5A est un exemple de mesure de type PPG (longueur d'onde de 810 $\pm 2$ nm), l'axe des ordonnées étant l'intensité du signal rétrodiffusé mesuré par le photodétecteur (unité arbitraire), l'axe des abscisses correspondant au temps (unité seconde). La courbe a) correspond à une mesure au niveau de la zone proximale (aorte) et la courbe b correspond à une mesure au niveau de la zone distale (poignet). Le temps de montée $\Delta t_1$ au niveau de la zone corporelle proximale est de 69 ms tandis que le temps de montée $\Delta t_2$ au niveau de la zone corporelle distale est de 59 ms.

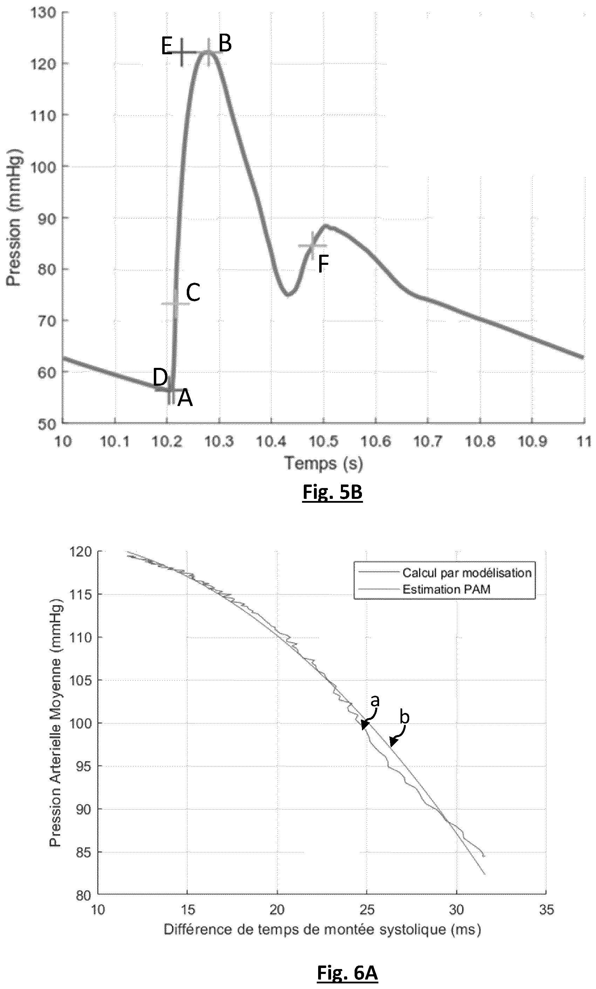

La détermination de chaque temps de montée peut être réalisée à partir de points fiduciels repérés sur chaque courbe représentant l'évolution d'une mesure en fonction du temps. On a représenté, sur la figure 5B, les points fiduciels suivant :

- un minimum de la mesure au cours de chaque période, chaque période correspondant à un battement cardiaque : cela permet de définir le début de la systole: point D. Le début de la systole peut également correspondre à une intersection entre une tangente à la courbe, par exemple au point auquel la pente est maximale, et un minimum de la courbe (point A).
- un maximum de la mesure au cours de chaque période, chaque période correspondant à un battement cardiaque : cela permet de définir la fin de la systole : point B. La fin de la systole peut également correspondre à une intersection entre une tangente à la courbe et un maximum de la courbe (point E).
- un point auquel la pente est maximal, entre le début et la fin de la systole : point C
- une intersection d'une tangente au point auquel la pente est maximale avec une droite parallèle à l'axe des abscisses passant par le maximum de la mesure au cours de la période considérée : point E;
- un point déterminé pour qu'une droite, passant par le minimum de la mesure (point A) et ledit point sépare la courbe correspondant à un battement en deux parties de surface égale point F.

Sur la figure 5B, l'axe abscisse correspond au temps (unité seconde) et l'axe des ordonnées correspond à une pression modélisée (unité mm Hg)

Etape 140 : comparaison

Au cours de cette étape, on compare les caractéristiques déterminées par la mesure proximale et la mesure distale. Par comparaison, on entend une différence ou un ratio.

Lorsque la caractéristique prise en compte est le temps de montée, la comparaison peut être une différence des temps de montée, correspondant à la VDMS précédemment décrite. Dans l'exemple donné en lien avec la figure 5A, la VDMS est de 10 ms.

Etape 150 : confrontation à un modèle

Au cours de cette étape, la comparaison résultant de l'étape 140 est confrontée à un modèle, de façon à estimer la pression artérielle. Le modèle peut être une fonction analytique, résultant d'une modélisation et/ou d'une calibration expérimentale. La définition du modèle fait l'objet d'une étape 90 mise en œuvre préalablement à l'étape 150. Selon une possibilité, le modèle est un algorithme d'intelligence artificielle à apprentissage supervisé, par exemple un réseau de neurones.

La figure 6A représente un premier modèle de calibration, permettant d'estimer la pression artérielle moyenne (axe des ordonnées - unité mmHg) en fonction de la VDMS. On observe de plus que la pression artérielle moyenne est une fonction monotone et décroissante de la VDMS. Une autre grandeur que la VDMS peut être utilisée, par exemple un ratio ou une différence entre les pentes des pics systoliques résultant des zones corporelles distale et proximale, sous réserve que la pression artérielle moyenne suive une fonction monotone en fonction de cette grandeur. Sur la figure 6A, on a représenté des points de modélisation (courbe a) ainsi qu'une interpolation polynomiale (courbe b). La modélisation a été effectuée en se basant sur un modèle cardiovasculaire, permettant de calculer les pressions et déformations des artères en différents points (Bednarek X. et al, « PPG sensor modelling for cardiovascular parameter estimation ». Virtual Physiological Human Conference 2022.). La variation locale de la pression et la variation locale des diamètres des vaisseaux permet de calculer, en plusieurs points, la durée de la montée systolique.

Sur la figure 6A, la pression artérielle moyenne PAM est reliée à la VDMS par un polynôme de degré 2 :

$$PAM = -0.062\,VDMS^2 + 0.796VDMS + 119.\,(1)$$

L'application du modèle à la VDMS permet d'estimer la pression artérielle moyenne :

$$AM = f(VDMS)\,(2)$$

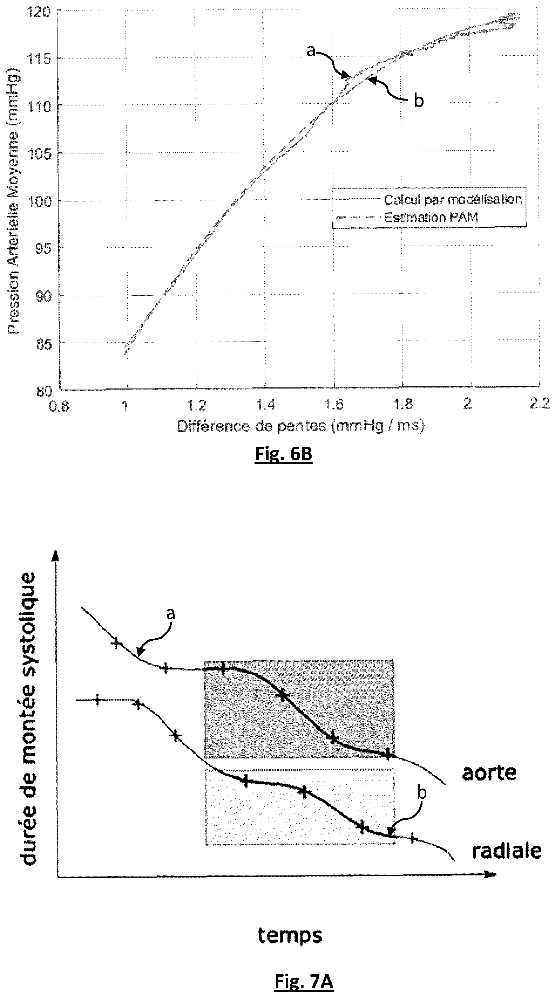

La figure 6B représente un deuxième modèle de calibration, permettant d'estimer la pression artérielle moyenne (axe des ordonnées - unité mmHg) en fonction d'une différence entre les pentes des pics systoliques résultant des zones corporelles distale et proximale. De même que sur la figure 6A, on a représenté, sur la figure 6B, des points de modélisation (courbe a) ainsi qu'une interpolation polynomiale (courbe b).

Sur la figure 6B on a représenté une différence de pentes moyennes sur la montée systolique. A la place de la pente moyenne, on pourrait également prendre en compte une pente maximale ou une combinaison de pentes locales. On observe de plus que la pression artérielle moyenne est une fonction monotone et croissante de cette variation de pente.

Le modèle utilisé dépend de chaque personne, ainsi que de la position de chaque point de mesure sur la personne. Il est possible de définir un modèle moyen, basé sur des modélisations et/ou des données expérimentales, pour une population. Un tel modèle, dit de population, peut être utilisé pour obtenir une première évaluation de la pression artérielle. Le modèle de population peut être individualisé en fonction de caractéristiques physiologiques de la personne : taille, poids, sexe...Le modèle peut également faire l'objet d'un recalage pour chaque individu, à partir d'une confrontation entre des mesures de la tension artérielle, résultant d'une méthode de référence, et de mesures de la VDMS.

Fréquence d'acquisition.

Les mesures, en chaque zone corporelle, sont effectuées selon une fréquence d'acquisition suffisamment élevée pour pouvoir détecter une VDMS faible, de valeur $VDMS_{min}$. Par exemple, en se basant sur une VDMS de 1ms, la fréquence d'acquisition F telle que :

$$F \geq \frac{4}{p \times VDMS_{min}} \ (3)$$

Où p désigne la précision de mesure souhaitée.
Par exemple,

$$\text{si } VDMS_{min} = 1 \text{ ms et } p = 0.05 \ (5\%), \ F \geq 80 \text{ kHz}.$$

$$\text{Si } VDMS_{min} = 10 \text{ ms et } p = 0.05 \ (5\%), \ F \geq 8 \text{ kHz}.$$

Les mesures du paramètre sont de préférence réalisées lors de différents battements successifs. Il est alors possible de déterminer, pour différentes mesures en une même zone corporelle, un temps de montée moyen (ou médian) $\overline{\Delta t_1}$ et $\overline{\Delta t_2}$. A partir de ces temps de montée moyens, on obtient une estimation de la $VDMS$ moyenne notée $\overline{VDMS}$. La figure 7A représente un tel mode de réalisation. Les courbes a) et b) correspondent respectivement aux temps de montée systolique (axe des ordonnées) à partir des mesures proximale et distales, l'axe des abscisses correspondant au temps. On peut procéder à une estimation de $\overline{VDMS}$ à partir de valeurs de $\overline{\Delta t_1}$ et $\overline{\Delta t_2}$ obtenues par moyennes glissantes.

On remarque que la mise en œuvre de la méthode ne suppose pas de synchronisation entre les mesures, comme le requièrent certaines méthodes mettant en œuvre une détermination de la pression artérielle basées sur une mesure de la vitesse d'onde de pouls.

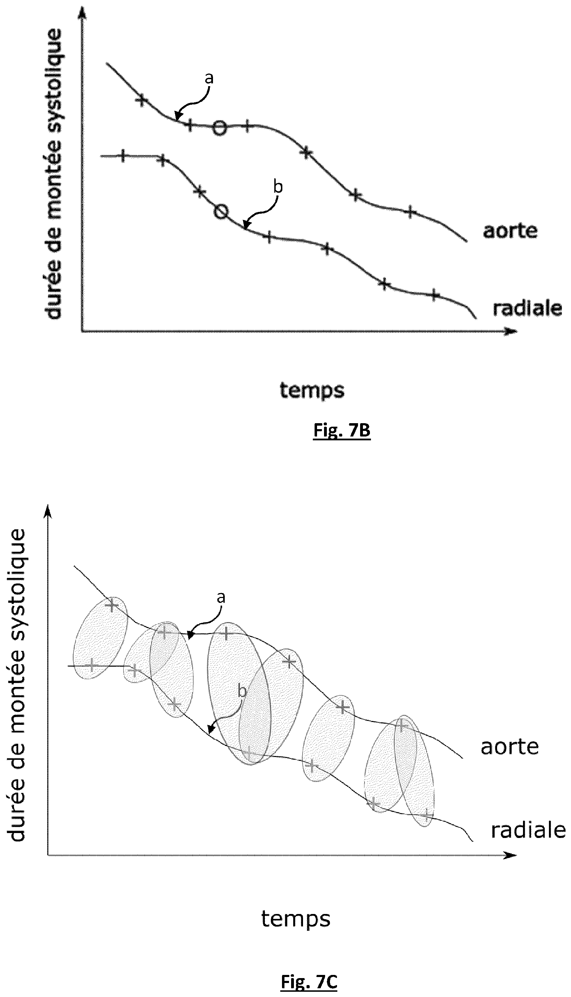

La figure 7B montre un mode de réalisation dans lequel on a déterminé différents temps de montée systoliques au niveau de la zone corporelle proximale et de la zone corporelle distale. Chaque temps de montée mesuré correspond à une croix sur chaque courbe. Il est ensuite possible d'effectuer des interpolations entre chaque temps de montée mesuré. Un tel mode de réalisation convient lorsque deux temps de montée successifs sont mesurés entre un nombre prédéterminé de battements. L'interpolation peut permettre de déterminer les temps de montée entre deux mesures. Sur la figure 7B, on a matérialisé chaque interpolation par un cercle.

La figure 7C représente un mode de réalisation dans lequel à partir de courbes montrant l'évolution, en fonction du temps, des temps de montée respectivement mesurés dans la zone proximale et la zone distale. Les points de mesures sont matérialisés par une croix. Il est possible de procéder à un appariement de points respectivement situés sur chaque courbe, de façon à identifier les points correspondant à un même battement. L'appariement peut être effectué en déterminant les points les plus proches sur chaque courbe. Il s'agit d'une interpolation simpliste : on estime la durée de montée systolique à un temps t, comme égale à la précédente mesurée. Le signal peut alors être traité par une électronique plus simple, les besoins en temps de calcul, mémoire, et en énergie sont réduits.

Bien que décrite en lien avec un dispositif 1, basé sur un capteur optique, l'invention peut s'appliquer à d'autres types de détecteurs, par exemple un détecteur acoustique, en particulier dans le domaine des ultrasons. On sait que l'échographie cardiaque peut également permettre d'accéder à des informations relatives à des variations pulsatiles de volumes sanguins. L'invention peut être appliquée à partir d'un signal d'échographie, par exemple une échographie Doppler. Le dispositif comporte au moins une source ultrasonore et au moins un capteur acoustique. Dans la pratique, on utilise généralement des transducteurs agissant à la fois en tant que source et détecteur.

L'invention peut également s'appliquer à l'échographie fœtale. Dans ce cas, chaque source et chaque détecteur ultrasonore est appliquée contre le fœtus, mais pas directement au contact de ce dernier, mais contre le ventre de la mère, ce dernier formant un milieu de propagation entre le dispositif et le fœtus.

L'invention peut également s'appliquer à un dispositif de type tonomètre comportant un capteur de pression appliqué au contact d'une zone corporelle.

L'invention peut également s'appliquer, de façon plus générale, à une mesure de tout paramètre variant de façon périodique sous l'effet d'une activité cardiaque ou respiratoire.

**Revendications**

1. Procédé de détermination d'une pression artérielle d'un utilisateur (*PAM*), en mesurant un paramètre suivant une variation monotone durant une systole, le procédé comportant :

   - a) au cours d'au moins un cycle cardiaque, mesure proximale du paramètre à une première distance du cœur et mesure distale du paramètre à une deuxième distance du cœur, la deuxième distance étant supérieure à la première distance ;
   - b) à partir de chaque mesure, estimation ($\Delta t_1$, $\Delta t_2$) d'une caractéristique représentative de la variation du paramètre durant la systole à chaque distance;
   - c) comparaison de chaque caractéristique (*VDMS*) ;
   - d) détermination de la pression artérielle (*PAM*),en fonction de la comparaison (*VDMS*) résultant de l'étape c), à l'aide d'un modèle de calibration (f) ;
   - le procédé étant **caractérisé en ce que** lors de l'étape b), la caractéristique représentative de la variation du paramètre durant la systole comporte

      • une durée ($\Delta t_1$, $\Delta t_2$) de la variation monotone du paramètre au cours de la systole
      • ou une pente de la variation monotone du paramètre au cours de la systole

2. Procédé selon l'une quelconque des revendications précédentes, dans lequel :

   - les étapes a) et b) sont mises en œuvre lors de plusieurs battements cardiaques ;
   - la caractéristique représentative de la variation du paramètre durant la systole comporte une durée moyenne $\overline{(\Delta t_1}, \overline{\Delta t_2)}$ de la variation monotone du paramètre au cours de la systole desdits battements cardiaque.

3. Procédé selon l'une quelconque des revendications 1 ou 2, dans lequel :

   - les étapes a) et b) sont mises en œuvre lors de plusieurs battements cardiaques ;
   - la caractéristique représentative de la variation du paramètre durant la systole comporte une pente moyenne ou maximale de la variation monotone du paramètre au cours de la systole desdits battements cardiaque.

4. Procédé selon l'une quelconque des revendications précédentes, dans lequel le paramètre est choisi parmi :

   - une intensité d'une lumière rétrodiffusée ou transmise par une artère ;
   - une intensité d'une onde acoustique réfléchie par une artère ;
   - une vibration induite par la systole ;
   - une impédance électrique induite par la systole.

5. Procédé selon l'une quelconque des revendications précédentes, dans lequel l'étape d) comporte le recours à un modèle de calibration analytique.

6. Procédé selon l'une quelconque des revendications 1 à 4, dans lequel le modèle de calibration est un algorithme d'intelligence artificielle supervisé.

7. Procédé selon l'une quelconque des revendications précédentes, dans lequel chaque mesure est réalisée en appliquant un capteur (20) face ou contre le corps de l'utilisateur.

8. Dispositif (1) d'estimation d'une pression artérielle d'un utilisateur, comportant :

   - deux capteurs (20), configurés pour être appliqués face à une artère de l'utilisateur, et configurés pour mesurer un paramètre de l'utilisateur, le paramètre suivant une variation monotone durant une systole;

- une unité de traitement (30), destinée à mettre en œuvre les étapes b), c) et d) d'un procédé selon l'une quelconque des revendications précédentes à partir du paramètre mesuré par chaque capteur (20).

9. Dispositif selon la revendication 8, dans lequel chaque capteur est choisi parmi : un capteur acoustique, un capteur optique, un capteur tonométrique ou un capteur d'impédance électrique, ou un capteur électromécanique.

**Fig. 1A**

**Fig. 1B**

**Fig. 1C**

**Fig. 2**

**Fig. 3**

**Fig. 4**

**Fig. 5A**

**Fig. 5B**

**Fig. 6A**

**Fig. 6B**

**Fig. 7A**

**Fig. 7B**

**Fig. 7C**

Europäisches
Patentamt

European
Patent Office

Office européen
des brevets

# RAPPORT DE RECHERCHE EUROPEENNE

Numéro de la demande

EP 25 17 2762

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | MUKKAMALA RAMAKRISHNA ET AL: "Toward Ubiquitous Blood Pressure Monitoring via Pulse Transit Time: Theory and Practice", IEEE TRANSACTIONS ON BIOMEDICAL ENGINEERING , vol. 62, no. 8 1 août 2015 (2015-08-01), pages 1879-1901, XP093222199, USA ISSN: 0018-9294, DOI: 10.1109/TBME.2015.2441951 Extrait de l'Internet: URL:https://ieeexplore.ieee.org/stampPDF/g etPDF.jsp?tp=&arnumber=7118672&ref=aHR0cHM 6Ly9pZWVleHBsb3JlLmllZWUub3JnL2RvY3VtZW50L zcxMTg2NzI= [extrait le 2024-11-08] * page 1880 - page 1883 * * page 1886 - page 1892 * * figures 6-8 * ----- | 1-9 | INV. A61B5/021 A61B5/00 |
| A | US 2013/066181 A1 (SCHECTER STUART O [US]) 14 mars 2013 (2013-03-14) * alinéa [0021] - alinéa [0026] * * alinéa [0044] * * alinéa [0069] - alinéa [0080] * * figures 5,6,8A-9 * ----- -/-- | 1-9 | DOMAINES TECHNIQUES RECHERCHES (IPC) A61B |

Le présent rapport a été établi pour toutes les revendications

| Lieu de la recherche | Date d'achèvement de la recherche | Examinateur |
|---|---|---|
| La Haye | 3 septembre 2025 | Van der Haegen, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

page 1 de 2

# EP 4 643 766 A1

Europäisches Patentamt
European Patent Office
Office européen des brevets

## RAPPORT DE RECHERCHE EUROPEENNE

Numéro de la demande

EP 25 17 2762

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | ADDISON PAUL S: "Slope Transit Time (STT): A Pulse Transit Time Proxy requiring Only a Single Signal Fiducial Point", IEEE TRANSACTIONS ON BIOMEDICAL ENGINEERING, IEEE, USA, vol. 63, no. 11, 1 novembre 2016 (2016-11-01), pages 2441-2444, XP011625964, ISSN: 0018-9294, DOI: 10.1109/TBME.2016.2528507 [extrait le 2016-10-18] * le document en entier * ----- | 1,4,7-9 | |
| A | US 2023/210492 A1 (JOSHI ROHAN [NL] ET AL) 6 juillet 2023 (2023-07-06) * alinéa [0010] * * alinéa [0023] * * alinéa [0130] - alinéa [0138] * * figures 1,3,7 * ----- | 1,4-9 | |
| | | | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| La Haye | 3 septembre 2025 | Van der Haegen, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

page 2 de 2

17

**ANNEXE AU RAPPORT DE RECHERCHE EUROPEENNE
RELATIF A LA DEMANDE DE BREVET EUROPEEN NO.**

EP 25 17 2762

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

03-09-2025

| Document brevet cité au rapport de recherche | Date de publication | Membre(s) de la famille de brevet(s) | | Date de publication |
|---|---|---|---|---|
| US 2013066181 A1 | 14-03-2013 | US | 2010049060 A1 | 25-02-2010 |
| | | US | 2013066181 A1 | 14-03-2013 |
| US 2023210492 A1 | 06-07-2023 | CN | 115776869 A | 10-03-2023 |
| | | EP | 3922175 A1 | 15-12-2021 |
| | | EP | 4164479 A1 | 19-04-2023 |
| | | JP | 7725506 B2 | 19-08-2025 |
| | | JP | 2023528682 A | 05-07-2023 |
| | | US | 2023210492 A1 | 06-07-2023 |
| | | WO | 2021250224 A1 | 16-12-2021 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **MUKKAMALA R et al.** Towards ubiquitous blood pressure monitoring via pulse transit time : theory and practice. *IEEE transactions on biomedical engineering*, 01 August 2015, vol. 62 (8)
- **OBATA Y** ; **MIZOGAMI M** ; **SINGH S** ; **NYHAN D** ; **BERKOWITZ DE** ; **STEPPAN J** ; **BARODKA V.** Ejection time: influence of hemodynamics and site of measurement in the arterial tree. *Hypertens Res.*, September 2017, vol. 40 (9), 811-818
- **BEDNAREK X. et al.** PPG sensor modelling for cardiovascular parameter estimation. *Virtual Physiological Human Conference*, 2022